# FASCICULE DE BREVET EUROPEEN

(11) **EP 1 460 189 B1**
(45) Date de publication et mention de la délivrance du brevet: **18.01.2012**
(21) Numéro de dépôt: 04290655.2
(22) Date de dépôt: 10.03.2004
(51) Int. Cl.: E03D 9/03, A61L 9/05, A61L 9/04, A61L 9/12

(54) **Dispositif hygiénique à diffusion progressive pour sanitaires**
Vorrichtung zur Abgabe von Duft- und Reinigungswirkstoffen in Sanitäreinrichtungen
Dispenser for sanitary appliances

(30) Priorité: 16.05.2003 FR 0305881; 19.03.2003 IT MI20030128 U
(43) Date de publication de la demande: 22.09.2004
(73) Titulaire: Bolton Manitoba SpA, 20124 Milano (IT)
(72) Inventeur: Luciani, Alain, 20054 Nova Milanese (MI) (IT); Oliva, Marco, 20155 Milano (IT); Stefanelli, Claudio, 00042 Anzio (Roma) (IT); Ferri, Michele, 20059 Vimercate (MI) (IT); Petrillo, Franco, 20141 Milano (IT)
(74) Mandataire: Coletti, Raimondo

(56) Documents cités:
- EP-A- 0 960 984
- WO-A-02/40792
- WO-A-03/027405
- DE-A- 19 901 457
- DE-A- 19 927 812
- US-A- 4 670 916
- US-B1- 6 662 380

## Description

L'invention a pour objet un dispositif hygiénique à diffusion progressive pour les sanitaires.

Dans les appareils sanitaires, comme les cuvettes de WC, l'efficacité du nettoyage et de la désinfection se dégrade rapidement, provoquant non seulement des conditions d'utilisation déplaisantes et anti-hygiéniques, mais aussi une dégradation de ces sanitaires due à la formation de calcaire et d'autres incrustations.

Les dispositifs hygiéniques qui s'installent à l'intérieur des toilettes sont très courants. Ils sont retenus à l'aide d'un système de suspension sur un bord de la cuvette des toilettes et ils sont partiellement balayés par l'eau de la chasse d'eau.

Ces dispositifs hygiéniques peuvent être du type «cage » comprenant un élément pourvu de fissures et/ou de trous qui permettent le passage de l'eau de la chasse d'eau, élément dans lequel se trouve un ou des composés chimiques actifs à l'état solide, ayant par exemple des fonctions détergentes et déodorantes.

Alternativement, des dispositifs hygiéniques comprenant un flacon contenant un ou des composés actifs chimiques sous forme liquide ou gel ont aussi été développés. Un flacon est disposé sur un distributeur qui est capable de libérer une quantité contrôlée de composés actifs à chaque flux de la chasse d'eau.

De tels dispositifs sont notamment décrits dans les demandes de brevet EP 538 957, WO 99 66140, WO 00 42261 et FR 00 10795.

Quel que soit le type de dispositif utilisé, le principe général est que, lors du déclenchement de la chasse d'eau, de l'eau libérée entre en contact avec les composés actifs chimiques et s'enrichit desdits composés qui lui confèrent des propriétés détergentes, décalcifiantes, désodorisantes et/ou désinfectantes en plus de son action nettoyante naturelle.

Cependant, les dispositifs hygiéniques connus pour sanitaires ne résolvent pas efficacement le problème de l'émanation dans l'environnement des mauvaises odeurs venant des toilettes.

En effet, l'éventuel effet parfumant et/ou désodorisant des composés actifs émis dans l'eau par les dispositifs hygiéniques connus n'est pas suffisant pour masquer les odeurs et, surtout, cet effet se maintient difficilement dans le temps, en particulier jusqu'au flux suivant de la chasse d'eau. Il est donc souvent nécessaire d'ajouter dans la pièce un désodorisant ou parfum d'intérieur.

Un autre dispositif hygiénique pour sanitaires est par exemple décrits dans WO02/40792 qui divulgue un dispositif hygiénique pour sanitaires avec un récipient comprenant des composés chimique actifs nettoyants susceptible d'entrer en contact avec l'eau de la chasse d'eau dans lesquels une substance volatile pour la distribution d'une odeur fraîche a été dissous.

US-A- 4670916 divulgue un dispositif hygiénique pour sanitaires avec un récipient comprenant des composés chimique actifs nettoyants susceptible d'entrer en contact avec l'eau de la chasse d'eau et un conteneur de distribution pur substances volatiles actionné lors du déplacement du siège de toilette dans une direction verticale. WO03/027405 divulgue un dispositif pour sanitaires comprenant une matrice servant de support à des substance volatiles parfumées qui ne sont pas capables d'entrer en contact avec l'eau de la chasse d'eau.

Il subsiste donc un besoin pour un dispositif hygiénique qui ait une action simultanément sur les surfaces des sanitaires et sur le masquage des odeurs dans le temps.

Un but de la présente invention est donc de proposer un dispositif hygiénique à diffusion progressive pour sanitaires qui contribue à résoudre les inconvénients mentionnés ci-dessus relatifs au masquage des odeurs dans le temps, tout en conservant ses propriétés de nettoyage des surfaces.

Par « propriétés de nettoyage », on entend dans la présente demande les propriétés nettoyantes, détergentes, décalcifiantes, et/ou désinfectantes.

Un autre but de l'invention est de proposer un dispositif hygiénique à diffusion progressive pour sanitaires, facile à utiliser et qui puisse être accroché à tous les types de sanitaires vendus dans le commerce.

Un autre but de l'invention est de proposer un dispositif hygiénique à diffusion progressive pour sanitaires qui soit facile et économique à réaliser et dont la fabrication ne nécessite pas une technologie complexe ou coûteuse.

La présente invention propose un dispositif hygiénique pour sanitaires tel que revendiqué.

Le terme « composés chimiques actifs nettoyants » désigne dans la présente demande un ou des composés chimiques actifs nettoyants, c'est-à-dire ayant des propriétés nettoyantes telles que définies ci-dessus, et susceptibles d'entrer en contact avec l'eau de la chasse d'eau. De tels composés peuvent également avoir en outre des propriétés parfumantes et/ou désodorisantes.

Le terme « substances volatiles » désigne dans la présente demande une ou des substances volatiles parfumées et/ou désodorisantes qui ne sont pas susceptibles d'entrer en contact avec l'eau de la chasse d'eau.

Des composés chimiques actifs nettoyants appropriés sont de tout type utilisé dans le domaine et connu de l'homme du métier, et peuvent être présents sous toute forme appropriée, en particulier sous forme solide, liquide ou gel tel que décrit précédemment. Un récipient les contenant pourra également être de tout type connu de l'homme du métier et utilisé dans le domaine, par exemple une « cage » ou un flacon. Un élément distributeur pourra être de tout type connu de l'homme du métier et utilisé dans le domaine, par exemple un tiroir téléscopique, ou des fentes présentes sur le récipient. Un élément distributeur permet de relâcher dans le flux de la chasse d'eau une quantité contrôlée ou non contrôlée de composés chimiques actifs nettoyants contenus dans un récipient.

Un récipient contenant les composés chimiques actifs nettoyants peut être amovible par rapport au reste du dispositif ; une fois vidé, un récipient peut donc être enlevé et remplacé par un récipient plein.

Une matrice servant de support aux substances volatiles peut être en tout matériau approprié pour libérer les substances volatiles, en particulier un semi-solide, un solide, un matériau caoutchouteux, un liquide ou un gel. Une matrice sera en contact avec l'air de la pièce dans lequel est placé le dispositif, par l'intermédiaire d'un moyen pour libérer en continu les substances volatiles dans l'air.

Ledit moyen pour libérer en continu les substances volatiles dans l'air peut prendre toute forme appropriée, et il peut s'agir en particulier d'une membrane semi-perméable, par exemple un film de polyéthylène perméable aux substances volatiles.

Ledit moyen peut être recouvert par un élément de protection amovible, en particulier un film qui sert de barrière externe, tel qu'un film plastique, par exemple un film en polyester. Un tel élément de protection protège la matrice servant de support aux substances volatiles en empêchant son contact avec l'air, et peut être retiré juste avant l'utilisation du dispositif. Une fois que cet élément de protection est retiré, la matrice est en contact avec l'air par l'intermédiaire du moyen pour libérer les substances volatiles dans l'air en continu et jusqu'à épuisement desdites substances volatiles, indépendamment de l'actionnement de la chasse d'eau.

Une membrane semi-perméable et le film protecteur correspondant peuvent être fabriqués par coextrusion.

Une matrice servant de support aux substances volatiles peut être contenue dans un récipient, qui peut être amovible par rapport au reste du dispositif.

Un tel récipient contenant une matrice peut être fixé, de manière amovible ou non, sur une partie d'un récipient contenant des composés chimiques actifs nettoyants qui n'est pas susceptible d'entrer en contact avec l'eau de la chasse d'eau, par exemple sur une partie supérieure de ce récipient. Cette fixation peut se faire en glissant et pressant le récipient contenant la matrice dans une ouverture de forme complémentaire présente sur le récipient contenant les composés chimiques actifs nettoyants.

Alternativement, le récipient contenant la matrice servant de support à des substances volatiles peut être fixé, de manière amovible ou non, à une autre partie du dispositif hygiénique selon l'invention qui n'est pas susceptible d'entrer en contact avec l'eau de la chasse d'eau.

D'une manière générale, dans un dispositif selon la présente invention, une matrice servant de support à des substances volatiles ne doit pas être susceptibles d'entrer en contact avec l'eau de la chasse d'eau. La libération des substances se fait indépendamment de l'actionnement de la chasse d'eau.

Un récipient contenant les composés chimiques actifs nettoyants et un récipient contenant la matrice servant de support à des substances volatiles peuvent donc être amovibles indépendamment l'un de l'autre, et peuvent donc être remplacés indépendamment l'un de l'autre.

Dans un mode de réalisation, une matrice servant de support à des substances volatiles peut être disposée directement dans ou sur un récipient contenant les composés chimiques actifs nettoyants, à un endroit non susceptibles d'entrer en contact avec l'eau de la chasse d'eau, par exemple dans des fentes ou rainures ouvertes sur l'extérieur disposées sur le récipient en question.

Si les composés chimiques actifs nettoyants sont épuisés avant les substances volatiles, ou inversement, le dispositif hygiénique à diffusion progressive selon l'invention peut encore être utilisé jusqu'à ce que les substances volatiles, ou inversement les composés chimiques actifs nettoyants, soient aussi épuisés, ou, si l'un des récipients est amovible, ce récipient peut être remplacé indépendamment du reste du dispositif.

En tout état de cause, les composés actifs chimiques nettoyants peuvent également avoir des propriétés parfumantes et/ou désodorisantes, complétant ainsi l'effet des substances volatiles.

Un dispositif selon l'invention peut comprendre un ou plusieurs récipients contenant des composés chimiques actifs nettoyants, par exemple deux récipients, chacun de ces récipients pouvant contenir des composés chimiques actifs nettoyants qui pourront être de nature et/ou de forme différente.

Un dispositif selon l'invention peut comprendre une ou plusieurs matrices servant de support à des substances volatiles, qui pourront être de nature différente.

Les caractéristiques et les avantages d'un dispositif hygiénique à diffusion progressive pour sanitaires selon l'invention seront précisés dans la description suivante d'exemples de mode de réalisation, exemples qui ne sont pas limitatifs, relativement aux figures annexées dans lesquelles :
- La figure 1 est une vue en perspective d'un premier mode de réalisation d'un dispositif hygiénique à diffusion progressive pour sanitaires selon l'invention;
- La figure 2 est une vue en perspective d'un autre mode de réalisation d'un dispositif hygiénique à diffusion progressive pour sanitaires selon l'invention, avec deux récipients pour composés chimiques actifs nettoyants;
- La figure 3 est une vue en perspective d'un autre mode de réalisation d'un dispositif hygiénique à diffusion progressive pour sanitaires selon l'invention.

Sur les figures sont représentés différents modes de réalisation d'un dispositif hygiénique 10 à diffusion progressive pour sanitaires selon l'invention, adapté pour être suspendu à l'intérieur des toilettes sur le bord de la cuvette 11 par l'intérieur d'un moyen 12, qui peut être de tout type connu de l'homme de l'art. Il peut s'agir par exemple d'une plaque flexible qui peut s'appliquer de façon universelle à cheval sur le bord supérieur de la cuvette des toilettes.

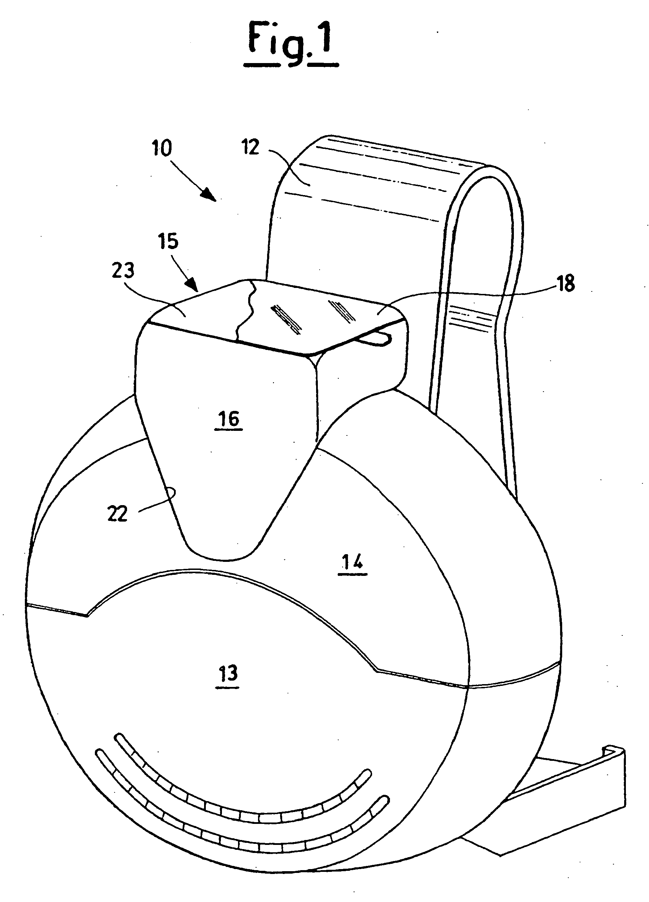

Dans la figure 1, le dispositif hygiénique à diffusion progressive 10 comprend un récipient 14 contenant des composés actifs chimiques nettoyants et un élément distributeur 13 pourvu d'ouvertures pour relâcher dans le flux de la chasse d'eau qui le balaye, une quantité de composés actifs chimiques nettoyants contenus dans le récipient 14. Le dispositif 10 comprend, de plus, un récipient 16 contenant une matrice servant de support à des substances volatiles, disposé de façon fixe ou amovible sur le récipient 14 dans une ouverture 22 de forme appropriée, et dont la face supérieure constitue un élément distributeur 15 pour la libération dans l'air des substances volatiles, élément qui dans ce mode de réalisation est une membrane semi-perméable 23. L'élément distributeur est ici recouvert par un élément de protection amovible 18.

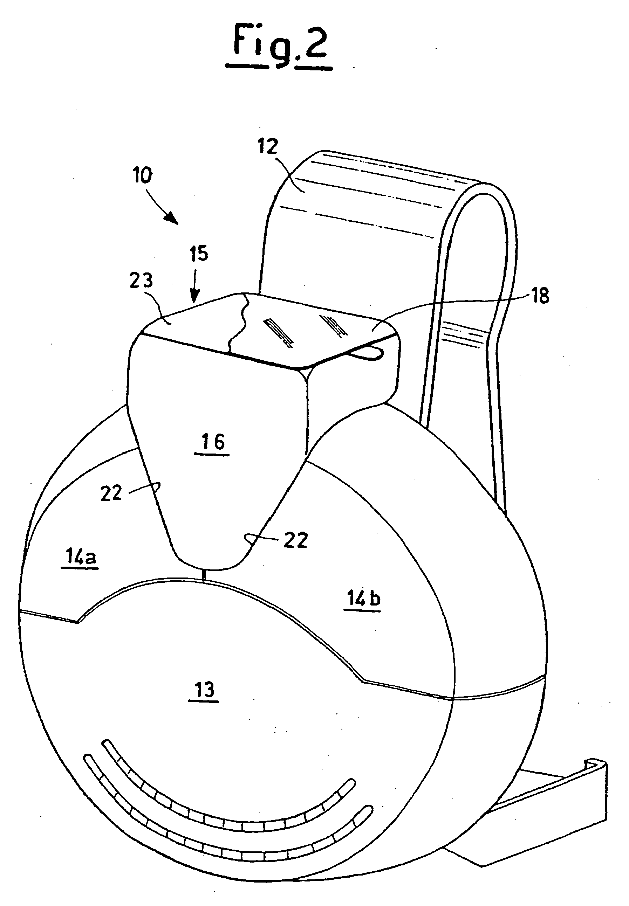

Dans la figure 2, le dispositif 10 selon l'invention comprend deux récipients 14a et 14b de composés actifs chimiques nettoyants. Les autres éléments sont semblables à ceux décrits en liaison avec la figure 1.

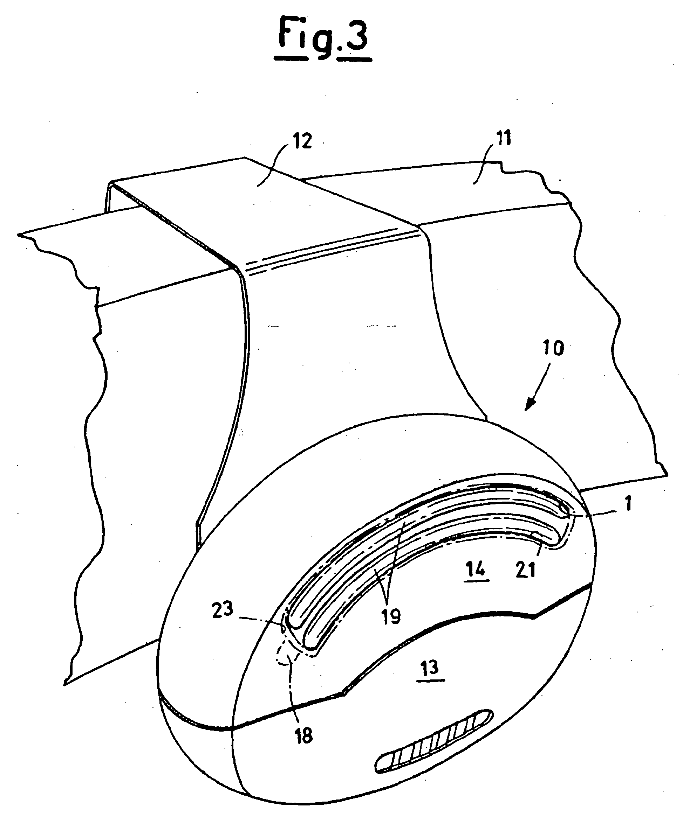

Dans la figure 3, le dispositif 10 selon l'invention est appliqué à un bord 11 de la cuvette des toilettes par un moyen de suspension 12. Le récipient 14 contenant les composés chimiques actifs nettoyants comprend des rainures 21 ouvertes vers l'extérieur à un endroit où le récipient n'est pas susceptible d'être en contact avec l'eau de la chasse d'eau. Dans ces rainures 21 est disposée une matrice 19 servant de support à des substances volatiles. Sur ces rainures contenant ladite matrice est disposée une membrane semi-perméable 23, et un élément de protection 18.

Le dispositif hygiénique à diffusion progressive pour sanitaires selon l'invention a l'avantage de fournir un intense parfum dans l'atmosphère et en particulier de parfumer et désodoriser de façon continue, indépendamment de l'action du flux de la chasse d'eau, et ceci en plus d'une action nettoyante. De plus, les substances volatiles et les composés chimiques actifs nettoyants sont conservés séparément et distribués respectivement dans l'air et dans le flux de la chasse d'eau.

## Revendications

1. Dispositif hygiénique (10) pour sanitaires, comprenant (i) un récipient (14) comprenant des composés chimiques actifs nettoyants capables d'entrer en contact avec l'eau de la chasse d'eau, (ii) un moyen (12) pour suspendre ledit dispositif à l'intérieur des toilettes sur le bord (11) de la cuvette des toilettes; (iii) un élément distributeur (13) pour relâcher dans le flux de la chasse d'eau une quantité de composés chimiques actifs nettoyants, **caractérisé** pour le fait que il comprend (iv) une matrice servant de support à des substances volatiles parfumées et/ou désodorisantes que ne sont pas capables d'entrer en contact avec l'eau de la chasse d'eau; et **caractérisé par le fait que** la matrice est contenue dans un récipient (16) qui est disposé dans une ouverture (22) de forme complémentaire présente sur le dispositif (10); et un des côtés du récipient (16) contenant la matrice constitue un moyen (15) pour libérer en continu dans l'air lesdites substances volatiles, ledit moyen (15) étant une membrane semi-perméable (23).

2. Dispositif selon la revendication 1 **caractérisé par le fait que** la membrane semi-perméable (23) est un film de polyéthylène.

3. Dispositif selon la revendication 1 **caractérisé** et par le fait que ledit moyen (15) est recouvert d'un élément de protection amovible (18).

4. Dispositif selon la revendication 2 **caractérisé par le fait que** le film de polyéthylène (23) est co-extrudé avec l'élément de protection (18) comprenant un film de polyester.

5. Dispositif selon la revendication 1 **caractérisé par le fait que** le récipient (16) comprenant la matrice est disposé, notamment par pression, sur une partie supérieure non en contact avec l'eau du récipient (14) comprenant des composés chimiques actifs nettoyants.

6. Dispositif selon la revendication 1 **caractérisé par le fait que** le récipient comprenant des composés chimiques actifs nettoyants est pourvu d'au moins une rainure (21) dans laquelle est disposée une matrice servant de support à des substances volatiles.

7. Dispositif selon la revendication 1 **caractérisé par le fait que** les composés chimiques actifs nettoyants sont présents sous forme de liquide ou de gel.

## Claims

1. Hygienic device (10) for sanitary appliances, comprising (i) a container (14) comprising active cleaning chemical compounds capable of coming into contact with the water of the flushing, (ii) a means (12) to suspend said device inside the toilet on the edge (11) of the toilet bowl; (iii) a dispensing element (13) to release into the flow of the flushing a quantity of active cleaning chemical compounds, **characterised in that** it comprises (iv) a matrix serving as a support to volatile perfumed and/or deodorising substances that are not capable of coming into contact with the water of the flushing; and **characterised in that** the matrix is contained in a container (16) which is arranged in an opening (22) of complementary shape found on the device (10); and one of the sides of the container (16) containing the matrix constitutes a means (15) to release continuously into the air said volatile substances, said means (15) being a semi-permeable membrane (23).

2. Device according to claim 1, **characterised in that** the semi-permeable membrane (23) is a polyethylene film.

3. Device according to claim 1, **characterised in that** said means (15) is covered by a removable protective element (18).

4. Device according to claim 2, **characterised in that** the polyethylene film (23) is co-extruded with the protective element (18) comprising a polyester film.

5. Device according to claim 1, **characterised in that** the container (16) comprising the matrix is arranged, in particular by pressure, on an upper part not in contact with the water of the container (14) comprising active cleaning chemical compounds.

6. Device according to claim 1, **characterised in that** the container comprising active cleaning chemical compounds is provided with at least one groove (21) in which a matrix serving as a support to volatile substances is arranged.

7. Device according to claim 1, **characterised in that** the active cleaning chemical compounds are found as liquid or gel.

## Patentansprüche

1. Vorrichtung (10) für Sanitäreinrichtungen, umfassend (i) einen Behälter (14), der chemische Reinigungswirkstoffe enthält, die in der Lage sind, mit dem Wasser im Spülkasten in Kontakt zu treten; (ii) Mittel (12), um die Vorrichtung ins Innere von Toiletten, und zwar an den Rand (11) der Toilettenschüssel zu hängen; (iii) ein Verteilerelement (13), um eine bestimmte Menge der chemischen Reinigungswirkstoffe in die Flüssigkeit im Innern des Wasserspülkastens abzugeben, **dadurch gekennzeichnet, dass** sie (iv) eine Matrize umfasst, die als Träger von flüchtigen Duft- und/oder Deodorantstoffen dient, die nicht mit dem Wasser des Spülkastens in Kontakt kommen können; und **dadurch gekennzeichnet, dass** die Matrize in einem Behälter (16) enthalten ist, der in einer zusätzlichen, auf der Vorrichtung (10) vorgesehenen Öffnung (22) angeordnet ist, wobei eine der Seiten des die Matrize haltenden Behälters (16) Mittel (15) zur Freisetzung der flüchtigen Stoffe in der Luft umfasst, wobei die Mittel (15) aus einer halb-durchlässigen Membran (23) bestehen.

2. Vorrichtung nach Anspruch 1, **dadurch gekennzeichnet, dass** die halbdurchlässige Membran (23) aus einer Polyethylenfolie besteht.

3. Vorrichtung nach Anspruch 1, **dadurch gekennzeichnet, dass** die Mittel (15) mit einem entfernbaren Schutzelement (18) bedeckt sind.

4. Vorrichtung nach Anspruch 2, **dadurch gekennzeichnet, dass** die Polyethylenfolie (23) mit dem Schutzelement (18) koextrudiert ist und eine Polyesterfolie umfasst.

5. Vorrichtung nach Anspruch 1, **dadurch gekennzeichnet, dass** der die Matrize umfassende Behälter (16) insbesondere mittels Druck auf einen oberen, nicht in Kontakt mit dem Wasser stehenden Teil des Behälters (14) angebracht wird, der die chemischen Reinigungswirkstoffe enthält.

6. Vorrichtung nach Anspruch 1, **dadurch gekennzeichnet, dass** der Behälter, der die chemischen Reinigungswirkstoffe enthält, mit mindestens einer Kerbe (21) versehen ist, in der eine Matrize angeordnet ist, die als Träger für die flüchtigen Stoffe dient.

7. Vorrichtung nach Anspruch 1, **dadurch gekennzeichnet, dass** die chemischen Reinigungswirkstoffe in Form einer Flüssigkeit oder eines Gels vorliegen.
